# EUROPEAN PATENT SPECIFICATION

(11) **EP 2 556 833 B1**
(45) Date of publication and mention of the grant of the patent: **26.07.2017**
(21) Application number: 11764967.3
(22) Date of filing: 07.04.2011
(51) Int. Cl.: A61K 33/14, A61K 33/00, A61K 9/20, A61K 9/08, A61P 25/28

(54) **PHARMACEUTICAL COMPOSITION FOR THE TREATMENT OF ALZHEIMER'S DISEASE, METHOD FOR PRODUCING SAME AND USE THEREOF**
PHARMAZEUTISCHE ZUSAMMENSETZUNG ZUR BEHANDLUNG VON MORBUS ALZHEIMER, VERFAHREN ZU IHRER HERSTELLUNG UND IHRE VERWENDUNG
COMPOSITION PHARMACEUTIQUE POUR LE TRAITEMENT DE LA MALADIE D'ALZHEIMER, PROCÉDÉ POUR SON OBTENTION ET UTILISATION

(30) Priority: 07.04.2010 BR PI1000970
(43) Date of publication of application: 13.02.2013
(73) Proprietor: Fundação Arnaldo Vieira De Carvalho, São Paulo - SP (BR)
(72) Inventor: DE SOUSA BUCK, Hudson, Mairiporã, CEP: 07600-000 SP (BR); ANDRADE NUNES, Marielza, Taubaté CEP: 12030-010 SP (BR)
(74) Representative: Aulich, Martin
(86) International application number: PCT/BR2011/000097
(87) International publication number: WO 2011/123916

(56) References cited:
- EP-A1- 0 234 733
- EP-A2- 0 289 204
- WO-A1-95/14481
- WO-A1-2004/107881
- WO-A1-2006/048773
- WO-A1-2010/098888
- WO-A1-2012/007387
- US-A- 6 160 018
- US-A1- 2004 110 837
- MACDONALD: "A feasibility and tolerability study of lithium in Alzheimer's disease", INTERNATIONAL JOURNAL OF GERIATRIC PSYCHIATRY, vol. 23, no. 7, July 2008 (2008-07), pages 704-711, XP002706350, ISSN: 0885-6230, DOI: 10.1002/GPS.1964
- POMARA NUNZIO: "Lithium treatment in Alzheimer's disease does not promote cognitive enhancement, but may exert long-term neuroprotective effects.", PSYCHOPHARMACOLOGY JUL 2009, vol. 205, no. 1, July 2009 (2009-07), pages 169-170, XP002706351, ISSN: 1432-2072
- JANOWSKY D S ET AL: "Minimally effective doses of conventional antipsychotic medications used to treat aggression, self-injurious and destructive behaviors in mentally retarded adults", JOURNAL OF CLINICAL PSYCHOPHARMACOLOGY,, vol. 25, no. 1, 1 February 2005 (2005-02-01), pages 19-25, XP009171486, ISSN: 0271-0749, DOI: 10.1097/01.JCP.0000150218.51433.7B
- DATABASE BIOSIS [Online] BIOSCIENCES INFORMATION SERVICE, PHILADELPHIA, PA, US; January 2013 (2013-01), NUNES MARIELZA ANDRADE ET AL: "Microdose Lithium Treatment Stabilized Cognitive Impairment in Patients with Alzheimer's Disease", XP002706352, Database accession no. PREV201300353899 & NUNES MARIELZA ANDRADE ET AL: "Microdose Lithium Treatment Stabilized Cognitive Impairment in Patients with Alzheimer's Disease", CURRENT ALZHEIMER RESEARCH, vol. 10, no. 1, January 2013 (2013-01), pages 104-107, ISSN: 1567-2050(print)
- C. J. PHIEL ET AL.: 'GSK-3a regulates production of Alzheimer's disease amyloid-beta peptides' NATURE vol. 423, no. 6938, 2003, pages 435 - 439, XP008161488
- M. SCHOU: 'Lithium treatment at 52' JOURNAL OF AFFECTIVE DISORDERS vol. 67, no. 1-3, 2001, pages 21 - 32, XP008161490
- M.S. ALLAGUI ET AL.: 'Long-term Exposure to low lithium concentrations stimulates proliferation, modifies stress protein expression pattern and enhances resistance to oxidative stress in SH-SY5Y cells' NEUROCHEM RES vol. 34, no. 3, 2009, pages 453 - 462, XP019685494

## Description

### Field of the invention

The present invention relates to the field of neurology and pharmacology. More specifically, the present invention describes the use of lithium compounds, especially salts, in microdoses for the treatment of Alzheimer's disease. The results show that the administration of lithium in microdoses is a known non-toxic alternative to the elderly for slowing or stopping the progress of the disease and the progressive cognitive deterioration, acting as an inhibitor of the enzyme GSK3β.

### Background of the invention

Alzheimer's disease (AD) is a neurodegenerative disease of great socioeconomic impact, accounting for about 50-60% of the total number of cases of dementia among people over 65. This disease affects about 1.5% of the population aged 65-69 years, 21% between 85-86, and 39% over 90 years, affecting approximately 15 million people worldwide. This disease is considered one of the major health problems due to the enormous impact to the individual, families, the healthcare system and society as a whole, since half of the patients are admitted to health institutions, but the rest receive treatment at home, involving family members, relatives and friends for their care. Invariably, monitoring the patient brings an enormous emotional, psychological and financial *stress* to families, since the treatment is expensive and the patient gradually loses their motor and learning functions, starting not to recognize close relatives. Scientists estimate that about 4 million people suffer from this disease and its incidence after age 65 doubles every 5 years. Moreover, in most industrialized countries, the population which is over 65 years old is a segment of the fastest growing population, expected to reach at least 19 million individuals by the year 2050. It is estimated that half of this quota may develop some form of AD.

The progressive degenerative process of cognitive and psychomotor functions, first described by the German pathologist Alois Alzheimer in 1907, lasts about 8.5 to 10 years, since the onset of clinical symptoms to death. The brain regions associated with higher mental functions, particularly the neocortex and the hippocampus, are the most affected ones by the biochemical changes resulting from the AD. Among the most evident causes of the genesis of the disease are the occurrence of extracellular deposition of peptide β-amyloid in senile platelets and the erratic formation of intracellular neurofibrils. This whole process results in loss of neuronal function and synaptic damage, with subsequent impairment of memory, motor coordination and reasoning, besides loss of cognitive ability and dementia.

The cytoskeleton of neurons consists of three main components: microtubules, microfilaments and intermediate filaments. The microtubules are hollow cylinders of 25 nm in diameter and whose length can reach more than 20 µm. The walls are formed by the aggregation of globular proteins of about 5 nm in diameter, the tubulins.

The tau proteins are a group of neuronal proteins that stabilize the microtubules under normal circumstances. Tau protein plays a key role in the regulation of microtubule dynamics of axonal transport and neurite outgrowth. All functions of Tau are modulated by phosphorylation at specific sites. Each isoform of tau protein is phosphorylated at a different site, under physiological conditions.

Numerous protein kinases phosphorylate the Tau protein *in vitro,* whereas *in situ* the list is much smaller. The protein kinase A, the kinases that regulate the affinity of the microtubule, the protein cyclin-dependent kinase 5 (cdk5) 1/p35 [p25] and the kinase 3-β glycogen synthase (GSK3) phosphorylate the Tau protein *in situ.* It is believed that the GSK3- β exerts a role in the phosphorylation regulation of the Tau protein in pathological conditions.

GSK3 is a regulatory enzyme highly expressed throughout the body and in large quantity in the brain, particularly the hippocampus. It is involved in the phosphorylation of numerous substrates, and regulates many physiological processes such as glycogen metabolism, gene expression, apoptosis, signal transduction and cell fate specification. It works in the metabolic signaling in structural proteins such as the tau proteins in the transcription factors. It is increased in the brains of patients with Alzheimer's disease, which leads to the hyperphosphorylation of the Tau protein and the instability of microtubules that accumulate in paired helical filaments. These, together with the amyloid β protein, lead to the degeneration with loss of the neuronal function and consequently to the Alzheimer's disease.

The enzyme GSK3-β is involved in almost all reactions that lead to the impairment of the brain in Alzheimer's disease. In transfected cells, as the expression of GSK3β increases there is an increase in the phosphorylation of tau and instability in the microtubule.

Lithium is the most studied inhibitor of the enzyme GSK3β. Approximately 50 years after the discovery of the antimanic properties of lithium in the treatment of bipolar disorders, it was identified that the GSK-3β was its main target. The mood-stabilizing properties of lithium, insulin-mimetic property of inhibitors of GSK-3β and the abnormal phosphorylation of the GSK-3β-dependent tau protein, with the production of the β-amyloid substance in Alzheimer's disease, have encouraged the search for potent and selective inhibitors of GSK-3β. So far, more than 30 inhibitors have been described.

The treatments for Alzheimer's disease which exist today include inhibitors of Cholinesterase, Rivastigmine and noncompetitive antagonists of NMDA receptors, Memantine. These drugs have relatively safe and mild symptomatic improvement. There are yet no drug that can stop the disease course and the progressive cognitive deterioration.

Considering the limitations of available drugs that are only symptomatic and can not meet the need to delay or even prevent Alzheimer's disease, the present invention provides a lithium for the treatment of this disease, since the same is a potent inhibitor of the enzyme GSK-3β, known to be involved in the pathogenesis of the disease, besides performing other neuroprotective functions, as seen before.

However, once the Lithium is toxic at high doses especially for the elderly population, we chose to use a presentation of Lithium in micro dose, as used in France, for the treatment of patients with depression and of those with proven efficacy at low doses.

Under the patent scope, some relevant documents were found, and they will be described below.

Document EP 0 234 733 describes the method for treating senile diseases, including Alzheimer's disease. The document describes the use of a compound of physiologically acceptable lithium for treating senile diseases. The accuracy of doses will depend on factors such as the patient weight and the severity of the condition, being suggested daily doses between 1 mg and 2000 mg. The present invention differs from said document for indicating the use of lithium salts in daily microdoses, lower values than those mentioned in the document, for being safer and producing satisfactory results.

Document WO 2007/132293 discloses a method of treating nervous system disorders using formulations containing daily dose. The document describes methods and formulations for treating CNS (category in which Alzheimer's disease is included) according to their metabolism in the body. It also describes that the total dose for treatment, prevention or management of the conditions related to the CNS commonly used ranges from 0.1 mg to 10,000 mg. The present invention differs from this document by using specifically lithium compounds for the specific treatment of Alzheimer's disease, data not mentioned in this document.

Document WO 1995/14481 describes the use of lithium compounds in combination with acetylcholinesterase inhibitors for the prevention of Alzheimer's disease. The present invention differs from said document by not comprising a combination with acetylcholinesterase inhibitors, but comprising only the use of lithium compounds in microdoses for the treatment of Alzheimer's disease.

Document EP 0 289 204 discloses general daily dosages of lithium salts ranging from 1 mg to 50 mg.

Dosage units of GSK-3 inhibitors such as lithium of 1 nanogram to 50 mg and daily dosages of 500 picogram per kg body weight are discussed in document US 2004 0 110 837.

Document WO 2006 048 773 shows daily administration of lithium doses of 10 microgram lithium per kg per rat to a rat, equivalent to dosage units of about 4 microgram.

In document WO 2004 107 881 pharmaceutical compositions with low microdoses of 20 to 500 microgram of lithium are disclosed.

The present invention provides an alternative solution to the problem of limitations of current treatments for Alzheimer's disease, so proven non-toxic, something that none of the documents mentioned herein made individually. Nor is there enough data in said documents so that, in combination, produce the effect achieved with the invention. After decades of use of lithium compounds at high doses, there are not yet known reports of substantial reduction of dosages of compounds containing lithium without loss of efficiency, as the present invention proposes.

Therefore, what is clear from the literature is that there were no documents found which anticipate or suggest the teachings of the present invention, so that the solution proposed here possesses novelty and inventive activity compared to the prior art.

### Summary of the invention

It is an object of the invention to provide therapeutic alternatives for the curative or prophylactic treatment of Alzheimer's disease.

In one aspect, being one of the objects of the invention, the present invention provides pharmaceutical compositions for the curative or prophylactic treatment of Alzheimer's disease, said compositions comprising compounds of lithium in microdoses.

In a preferred aspect, being another of the objects of the invention, it is provided a pharmaceutical composition for use in the curative or prophylactic treatment of Alzheimer's disease according to claim 1.

Preferably said lithium salt is lithium gluconate, lithium carbonate or combinations thereof.

It is another object of the present invention a process for the preparation of a pharmaceutical composition for the curative or prophylactic treatment of Alzheimer's disease. Said process comprises formulating a pharmaceutically acceptable carrier with at least one active ingredient comprising between x and y (concentration) of compounds selected from lithium, or combinations thereof. A corresponding process is subject of claim 2.

It is another object of the invention to use lithium compounds in microdoses in the preparation of medicaments for the curative or prophylactic treatment of Alzheimer's disease such as the pharmaceutical composition described above.

These and other objects of the invention will be immediately appreciated by those skilled in the art and by companies with interests in the segment, and will be described in sufficient details to be reproduced in the following description.

### Brief description of the drawings

Figure 1 shows the distribution by age and gender of patients with Alzheimer's symptoms. The X axis represents the age in years, and the Y axis represents the number of patients. The results in black represent male patients, and results in white represent female patients.
Figure 2 shows the distribution by age and gender of patients participating in the research. The X axis represents the age in years, and the Y axis represents the number of patients. The results in black represent male patients, and results in white represent female patients.
Figure 3 represents the score obtained by the research participants in the four quarters prior to entering the experimental protocol, in which: (M) MMSE score; (T) time in quarters; (L) results from the group treated with lithium; and (C) results from the control group.
Figure 4 shows the distribution of participants in the age and gender protocol (p = 0.3860), in which: (M) males; (F) females; and the Y axis represents the age in years.
Figure 5 shows the comparison between the age groups of female participants of groups Treated and Control (p = 0.7582), in which: (FT) Treated Females; (FC) Female Control; and the Y axis represents the age in years.
Figure 6 shows the comparison between the age groups of male participants of groups Treated and Control (p =0.6162), in which: (MT) Treated Males; (MC) Male Control; and the Y axis represents the age in years.
Figure 7 shows the comparison between the age groups of research participants of the groups treated and control (p = 0.4278), in which (T) Treated; (C) Control; and the Y axis represents the age in years.
Figure 8 shows the performance of participants of groups the control and Lithium in MMSE during the duration of the experimental protocol. Statistical comparisons by two-way ANOVA followed by Bonferroni test. For: (*) P<0.01; (**) P<0.05; (a) P<0.001; (M) performance in MMSE; and (T) Time in quarters.
Figure 9 shows the results of MMSE scores of the patients during the six quarters of the study analyzed by gender (F1.317= 0.5853, p=0.4448; F 317 = 0.0.1545, p=0.9786), in which: (M) performance in MMSE; (T) Time in quarters; (MT) Treated Males; (FT) Treated Females.
Figures 10, 11 and 12 show the evolution of MMSE in the groups control and Lithium, analyzed by age, in which Figure 10 represents the group between 61 and 70 years old, Figure 11 represents the group between 71 and 80 years old, and Figure 12 represents the group over 80 years old. We can see a significant difference between the groups aged 70 to 80 years and over 80 years old. We also see that there is no difference in the age group from 60 to 70 years.

### Detailed description of the invention

The present invention provides therapeutic alternatives for the curative or prophylactic treatment of Alzheimer's disease. In one aspect, the present invention provides pharmaceutical compositions for the curative or prophylactic treatment of Alzheimer's disease, said compositions comprising compounds of lithium in microdoses. In a preferred aspect, it is provided a pharmaceutical composition for the curative or prophylactic treatment of Alzheimer's disease comprising:
a) a pharmaceutically acceptable carrier; and
b) up to 0.28 mg (daily dose) of at least one active ingredient selected from the group comprising lithium salts or combinations thereof.

Preferably said lithium salt is lithium gluconate, lithium carbonate or combinations thereof.

The present invention also provides a process for the preparation of a pharmaceutical composition for the curative or prophylactic treatment of Alzheimer's disease. Said process comprises formulating a pharmaceutically acceptable carrier with at least one active ingredient comprising between up to 0.28 mg (daily dose) of compounds selected from lithium, or combinations thereof. Preferably, said lithium compounds are in the form of sublingual globules.

The present invention also provides the use of lithium compounds in microdoses in the preparation of medicaments for the curative or prophylactic treatment of Alzheimer's disease.

The examples shown below are intended only to exemplify one of many ways of performing the invention, but without limiting the scope thereof.

### PHARMACEUTICAL COMPOSITION FOR THE CURATIVE OR PROPHYLACTIC TREATMENT OF ALZHEIMER'S DISEASE

The pharmaceutical composition for the curative or prophylactic treatment of Alzheimer's disease of the present invention is characterized in that it comprises:
a) a pharmaceutically acceptable carrier; and
b) up to 0.28 mg (daily dose) of at least one active ingredient selected from the group comprising lithium salts or combinations thereof.

### PHARMACEUTICALLY ACCEPTABLE CARRIER

The vehicle of the present invention comprises any pharmaceutically acceptable carrier which may be, but is not limited to tablets, capsules or drops.

### LITHIUM SALTS

The lithium salt to be used may comprise, but is not limited to lithium gluconate or lithium carbonate.

### PROCESS FOR THE PREPARATION OF A PHARMACEUTICAL COMPOSITION FOR THE CURATIVE OR PROPHYLACTIC TREATMENT OF ALZHEIMER'S DISEASE

The process for preparing a pharmaceutical composition for the curative or prophylactic treatment of Alzheimer's disease of the present invention comprises means for producing said pharmaceutical composition which may be, but not limited to means for producing tablets, capsules or drops.

### EXAMPLE 1. PREFERRED EMBODIMENT

### MATERIAL AND METHODS

The patients were selected after approval by the Ethics Committee on Human Research.

### INCLUSION CRITERIA

Patients clinically diagnosed with Alzheimer's disease by the DSM-IV and NINCDS-ADRDA criteria, and under treatment. The patients were selected by the Mini Mental State Examination (MMSE), which is a questionnaire asked to the patient at each visit, which has a maximum score of 30 points. The Mini-Mental State Examination (MMSE) was designed to be a clinical practice assessment of change in the cognitive status in geriatric patients (Folstein *et al,* 1975). It examines temporal and spatial orientation, short-term memory (or immediate attention) and recall, calculation, apraxia, and language and visual-spatial skills. It can be used as a screening test for cognitive impairment or as a cognitive assessment of bedside. The Portuguese version of the MMSE (Bertolucci *et al,* 1994) was considered reliable and adequate for screening and monitoring of ambulatory elderly (Lawrence *et al,* 2008).

The MMSE includes 11 items, divided into 2 sections. The first requires verbal answers to questions of orientation, memory and attention, the second to questions of reading and writing skills, and it evaluates the skills of appointment, follow verbal and written commands, writing a sentence and copying a drawing (polygons) (Folstein *et al*, 1975). The cutoff point most often used to indicate a cognitive impairment that deserves further investigation is 24. Some authors suggest 25 to increase the sensitivity to mild dementia (Kay *et al*, 1985). The cutoff point is often adjusted for the education level because a single cut can lose cases among persons with more education, and generate false positives among those with less education. Some authors have suggested that the cutoff 24 proved to be excellent for people with education above 9 years, while the cutoff 17 was great for those with less education (Bravo, Hébert, 1997). Some Brazilian authors studied the measuring characteristics of the scale. Thus, to evaluate the cognitive performance of a population seeking medical screening service of a hospital, Bertolucci applied the MMSE in 530 individuals. We found distinct cutoff points for the diagnosis of cognitive decline, according to the level of education: 13 for illiterates, 18 for low and middle levels of education and 26 for high level of education, with a sensitivity of 82.4%, 75.6% and 80%, and specificity of 97.5%, 96.6% and 95.6%, respectively (Bertolucci *et al*, 1994).

The MMSE was the parameter chosen for inclusion and monitoring of patients of the search for being a test performed on all patients in this clinic and for being a cognitive assessment scale that is practical and useful in the investigation of patients with dementia risk, as in the case of elderly (Almeida, 1998), and it is prescriptive by use and the criteria for dispensing medications adopted by the Ministry of Health. The fact that all medical professionals involved in the evaluation of patients are fully trained and qualified in applying the MMSE was also relevant for this choice. The inclusion of a second test could generate a methodological error that is not measurable. The patients were diagnosed by a neurologist, psychiatrist or geriatrician as suffering from dementia due to Alzheimer's disease, according to the criteria previously mentioned. Patients must present MMSE score equal to or greater than 12 for patients over 4 years of education and greater than or equal to 9 for patients with up to 4 years of education. (Almeida, 1998).

These tests are performed routinely in the clinic by the attending physician, and they are recorded in the patients' records.

### EXCLUSION CRITERIA

The patients with Bipolar Disorder and under treatment or who were treated with lithium, with MMSE score below 12 with education higher than 4 years and 9 with education lower than 4 years were excluded, because they present an advanced stage of the disease. Patients under 60 years of age were also excluded.

### IMPLEMENTATION OF THE SEARCH

We initially considered the medical records of 346 patients who were undergoing treatment at the time of the beginning of the research. 166 patients were selected after the evaluation of the criteria for inclusion and exclusion in private interview with each one. The Terms of Free and Clear Consent have been duly signed. Remained in the study 75 women and 40 men, and it was noted that two patients did not have the scores required to participate in the search. Then, 73 women and 40 men remained in our study. The sample used was analyzed using a formula to compare two means from different populations (Program DIMAM 1.0) (Arango, 2005) and had an allowed sampling error of 2.4; a standard deviation of 4.9 with a testing power of 95%. FORMULA n= (Critical value 2 x δ2) / d2 n= (1.962 X 4.92) / 0.82 n= 115

In this preferred embodiment of the invention, the drug tested was the lithium gluconate in a dose of 8.140 mg in 2 mL of solution, which corresponds to 0.28 mg or 0.04 mMol of the metal administered sublingually. Two presentations were used: one containing the lithium gluconate and other containing placebo (anhydrous glucose and purified water). Both presentations were differentiated by a label existing in a group, placebo or lithium, unknown by researchers and by the executors of the MMSE. The patients were selected in a blinded fashion in both groups, with and without stamp by attendance order, being the first with the seal, alternating until the last patient. Formulations were also evaluated with lithium carbonate, respecting the dose of 0.28 mg of metal per dose. As 1000 mg of lithium gluconate have 4.95 mmol, 8.140 mg have 0.04 mMol 1000 mg of lithium carbonate and have 27 mMol in order to give 0.04 mMol; the administration dose was 1.5 mg. This dose of lithium carbonate is not commercially available, having the sublingual globules of lithium carbonate at a dose of 1.5 mg been developed and used. The patients were in consultation at least every 3 months, when the new MMSE was performed by the attending physician. The patients were followed every 3 months, with the delivery of medicines and their interview at the time, noting the complications in a spreadsheet along with the results of the MMSE, performed by doctors of the service. Statistical analyzes were performed in retrospective of homogeneity of the sample with respect to age, gender, initial MMSE score, MMSE regarding developments during the year preceding the beginning of the research, about the time of diagnosis of probable Alzheimer's disease and regarding the other medications with possible interference with lithium used by patients.

### STATISTICS

The software used for the analysis was the GraphPad Prism 3.0.

The assessment of the homogeneity of the sample was performed with Student's t test for unpaired samples. The statistical comparison between the MMSE scores of lithium and control groups was performed by the two-way analysis of variance (ANOVA). In the ANOVA, the treatment factors (control vs. Lithium) and the time (MMSE score over time) were considered. ANOVA was performed subsequent to the Bonferroni test. For the comparison of the time of diagnosis of probable AD in control and Lithium groups we used the Fisher exact test. For the comparison of the use of drugs that may have influenced the outcome of the use of lithium we used the Chi square test. Differences were considered significant at p<0.05. 39

### RESULTS

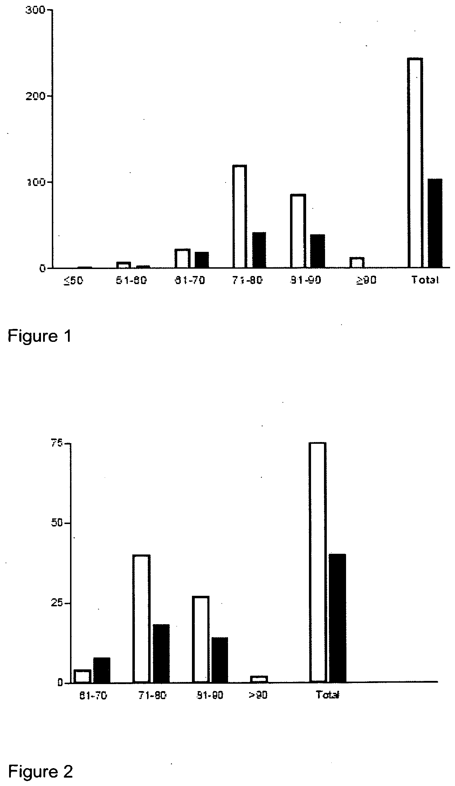

Analysis of the total population of the clinic and the study population: The clinic patients were admitted to for treatment based on DSM-IV and NINCDS-ADRDA criteria. The characteristics (age, gender and MMSE) of the total population of the clinic where the study was conducted were determined through the analysis of the medical records. Patients were grouped by age and gender. From a total of 346 patients, 70% were female and 30% male. It was observed that 82% of the patients were in the range between 71 and 90 years old (Figure 1). The participants were 75 women and 40 men aged between 51 and 92 years, and 86% were between 71 and 90 years, and 65% were female and 35% male (Figure 2).

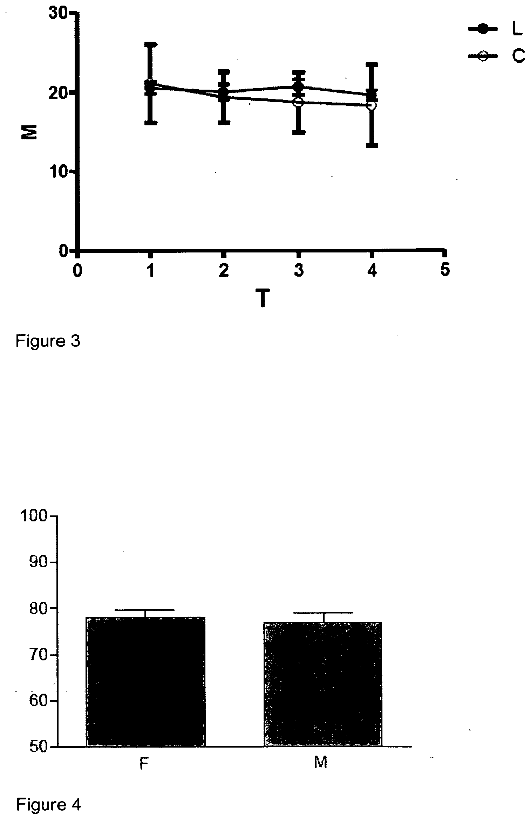

MMSE evaluation of the subjects of the study before the initiation of the treatment. To evaluate the performance of the selected groups in the MMSE, the score obtained was evaluated in the four quarters prior to the start of the study. The results showed that both control and treated groups did not present significant differences between the treatment and time factors (F1, 301=2.185 p=0.1404, F3, 301=1.800 p=0.1472, respectively, Figure 3). There were also no significant differences with the Bonferroni test.

There was no significant difference between the mean age of women and men participating in the protocol (78.00 ± 0.8165 and 76.80 ± 1092, p=0.3860, respectively) (Figure 4).

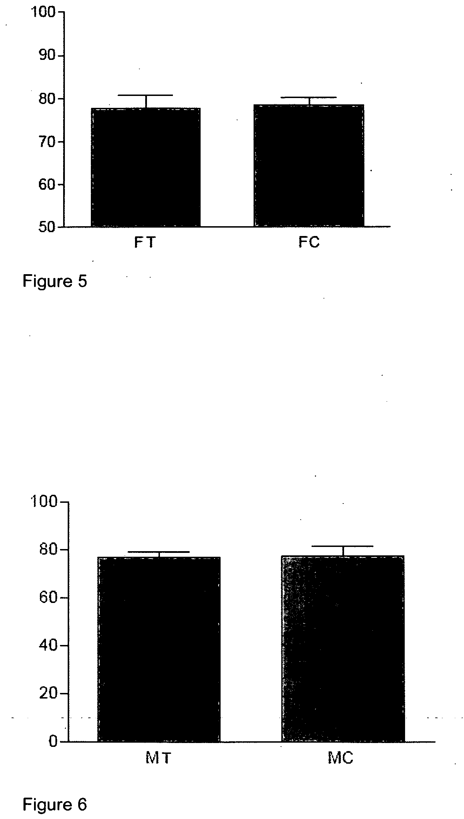

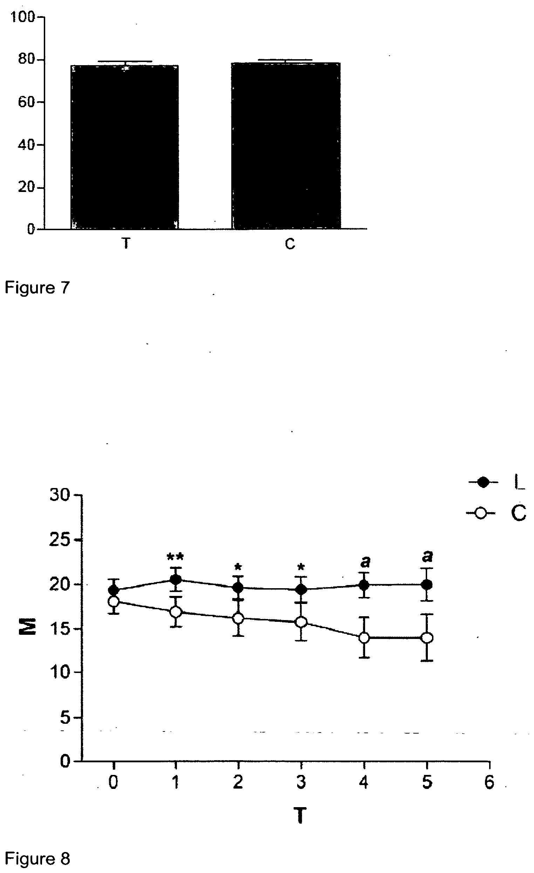

No significant differences were observed when comparing age from control and Lithium groups separated by gender, both male (Control= 77.30 ± 1.833, Lithium 76.61 ± 1279, P=0.7582) and female (Control 78.58 ± 0.8464; Lithium 77.78 ± 1.451, p=0.6162) (figures 5 and 6). A comparison of age between the control and treated groups without division by gender also did not present differences between the groups (Treated 77.40 ± 0.9769, Control 78.39 ± 0.7656, P = 0.4278; Figure 7).

There were no significant differences (Fisher's exact test) between control and treated groups for individuals with diagnosis time of Alzheimer's disease below six years, and for a diagnosis over six years before the start of the protocol (Table 1).

**Table 1. Comparison between the time of diagnosis of probable Alzheimer's disease between lithium and Control groups (number of individuals).**

| | Start <6 years | Start > 6 years |
|---|---|---|
| Lithium | 45 | 8 |
| Control | 43 | 8 |

In order to evaluate the possible influence of some drugs that can act directly or indirectly in the treatment of AD, we evaluated the distribution of patients using these drugs between Control and Lithium groups. We evaluated the users of the following medications: cholinesterase inhibitors, NMDA receptor inhibitors, inhibitors of angiotensin and hypoglycemic converting enzyme. No significant differences were observed between Control and Lithium groups for any of the drugs (Table 2).

**Table 2. Comparison between the various drugs used by patients in both groups: Lithium and Control.**

| | Acelil-cholinesterase inhibitors | Inhibitors of the NMDA receptor | Inhibitors of Angiotensin Converting Enzyme | Hypoglycaemic |
|---|---|---|---|---|
| Lithium | 26 | 45 | 19 | 8 |
| Control | 22 | 46 | 15 | 7 |

### EFFECT OF TREATMENT WITH MICRODOSES OF LITHIUM ON THE MEMORY OF PATIENTS DIAGNOSED WITH ALZHEIMER'S DISEASE

In evaluating the scores obtained in quarterly reviews by the MMSE, a significant decrease in the MMSE scores obtained in the control group was observed, as opposed to the maintenance of these scores by the group treated with microdoses of lithium. The two-way variance analysis showed significant difference between the control and Lithium groups in the treatment factor (F1, 614 = 65.81 p<0.0001). There was no significant difference in the time factor (F5, 614= 1.695 p=0.1336; Figure 8). Subsequent analysis by the Bonferroni procedure showed that the MMSE performed immediately before the inclusion in the experimental protocol (quarter 0) did not show significant difference between the control and Lithium groups (18±0.67, n=57 and 19±0.63, n=56, P=0.17, respectively). From the first quarter on it was observed a significant difference between the control and lithium groups (16.91 ± 0.83 vs 20.57 ± 0.66, P <0.01, respectively) which was extended until the end of the study (Quarter 5; control = 14 ± 1.326 and Lithium = 20 ± 0.9, P <0.001).

### ANALYSIS OF THE INFLUENCE OF GENDER OF PARTICIPANTS ON TREATMENT WITH LITHIUM

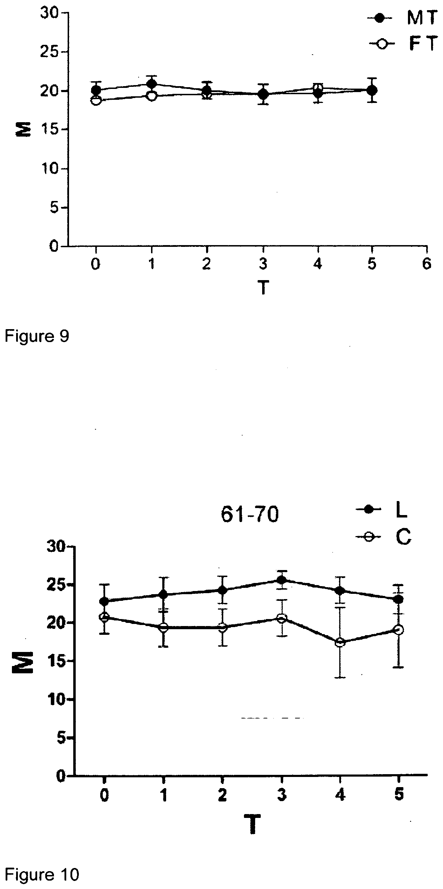

To determine whether gender could influence the effects of treatment, the scores obtained of men and women in the treated group were compared. There were no significant differences in treatment and time factors (F1,317= 0.5853, p=0.4448 and F 0.0.1545 = 5.317, p = 0.9786, respectively, Figure 9). There were also no significant differences in the subsequent analysis by the Bonferroni procedure.

### EFFECT OF TREATMENT WITH LITHIUM IN GROUPS DIVIDED BY AGE

To determine whether the efficacy of treatment with lithium may be affected by the age group of the participants, the groups were divided into ranges of 61 to 70 years, 71 to 80 years and above 80 years. It is noteworthy that the power of this analysis is compromised by the sample size, due to segmentation by age of the total group.

61-70 years: The comparison made between Control and Lithium groups shows significant differences in the treatment factor (F1, 62 = 8.487, p = 0.0053) but not in the time factor (F5, 62 = 0.1849, p = 0.9670; Figure 10) characterized by a decrease in the MMSE scores obtained from participants in the control group. Subsequent analysis by the Bonferroni test revealed no significant differences.

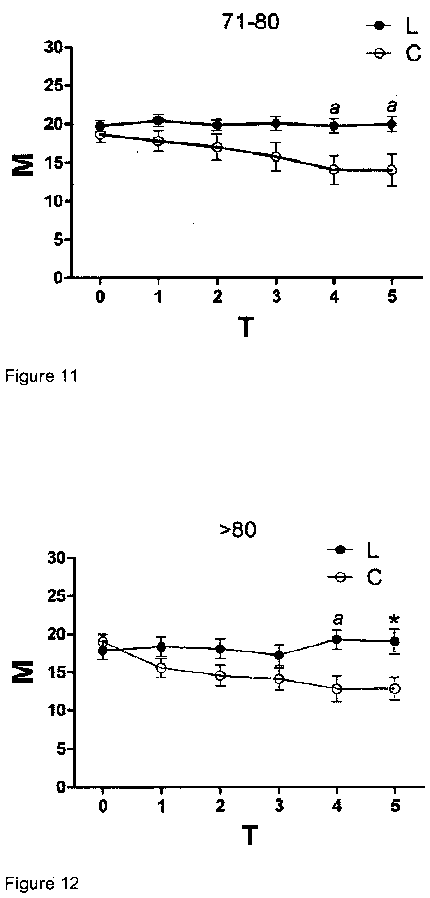

71-80 years: In this age group there was significant difference in the treatment factor (F1, 302= 29.6, p<0.0001), but no statistically significant difference was observed in the time factor (F5, 302= 1.410, p=0.2744. Figure 11). The difference in the treatment factor was characterized by the decrease in the MMSE scores in the control group, accompanied by stable maintenance of these values in the group treated with lithium. Subsequent analysis by the Bonferroni test showed significant decrease of these scores in the control group in assessments of quarters four and five.

>80 years: Similarly to that obtained in the age group of 71-80 years of age, participants over 80 years old in the group treated with lithium had better scores on the MMSE compared to control subjects, which showed a significant reduction of this score (treatment factor F1,225= 19.53, p<0.0001). There was no significant difference in the time factor (F5,225= 1.108, p=0.3571; Figure 12). The analysis by the Bonferroni test showed significant decrease of these MMSE scores in the control group in assessments of quarters four and five.

### CONCLUSIONS

The results obtained indicate that the Lithium in microdoses seems to preserve the memory of individuals with Alzheimer's disease. Furthermore, the response to the treatment seems to be more significant in patients over 70 years. The biochemical and histological changes involved in this process still need to be determined. By presenting little risk and low cost at the doses studied, the lithium can be of great therapeutic value to the stabilization of the cognitive processes of individuals with Alzheimer's disease.

Those skilled in the art will appreciate the teachings showed herein and can reproduce the invention in the modalities shown and in other variants falling within the scope of the appended claims.

## Claims

1. A pharmaceutical composition for use in the curative or prophylactic treatment of Alzheimer's disease **characterized in that** it comprises:
a) a pharmaceutical effective microdose of lithium salt, wherein said microdose is up to 0.28 mg of lithium salt; and
b) a pharmaceutically acceptable carrier.

2. A pharmaceutical composition for the use according to claim 1, wherein said lithium salt form is gluconate lithium, lithium carbonate or combinations thereof.

3. A process for preparing a pharmaceutical composition according to anyone of claims 1 or 2 **characterized in that** it comprises the step of mixing gluconate lithium, lithium carbonate or combinations thereof with a pharmaceutically acceptable carrier.

4. The use of a pharmaceutical composition according to anyone of claims 1 or 2 in the preparation of a medicament for the curative or prophylactic treatment of Alzheimer's disease.

5. The use, according to claim 4, wherein said treatment consists of the daily dosage of said medicament.

## Patentansprüche

1. Pharmazeutische Zusammensetzung zur Verwendung bei der kurativen oder prophylaktischen Behandlung von Alzheimer-Krankheit, **dadurch gekennzeichnet, dass** sie Folgendes umfasst:
a) eine pharmazeutisch wirksame Mikrodosis an Lithiumsalz, wobei die Mikrodosis bis zu 0,28 mg Lithiumsalz beträgt, und
b) einen pharmazeutisch unbedenklichen Träger.

2. Pharmazeutische Zusammensetzung zur Verwendung nach Anspruch 1, wobei es sich bei der Lithiumsalzform um Gluconat-Lithium, Lithiumcarbonat oder Kombinationen davon handelt.

3. Verfahren zur Herstellung einer pharmazeutischen Zusammensetzung nach Anspruch 1 oder 2, **dadurch gekennzeichnet, dass** es den Schritt des Mischens von Gluconat-Lithium, Lithiumcarbonat oder Kombinationen davon mit einem pharmazeutisch unbedenklichen Träger umfasst.

4. Verwendung einer pharmazeutischen Zusammensetzung nach Anspruch 1 oder 2 bei der Herstellung eines Medikaments zur kurativen oder prophylaktischen Behandlung von Alzheimer-Krankheit.

5. Verwendung nach Anspruch 4, wobei die Behandlung aus der täglichen Verabreichung einer Dosis des Medikaments besteht.

## Revendications

1. Composition pharmaceutique pour utilisation dans le traitement prophylactique ou curatif de la maladie d'Alzheimer, **caractérisée en ce qu'**elle comprend :
a) une microdose pharmaceutiquement active de sel de lithium, où ladite microdose peut atteindre 0,28 mg de sel de lithium ; et
b) un vecteur pharmaceutiquement acceptable.

2. Composition pharmaceutique pour utilisation selon la revendication 1, où la forme dudit sel de lithium est le gluconate de lithium, le carbonate de lithium ou leurs combinaisons.

3. Procédé d'élaboration d'une composition pharmaceutique selon l'une quelconque des revendications 1 ou 2, **caractérisée en ce qu'**elle comprend l'étape de mélangeage du gluconate de lithium, du carbonate de lithium ou de leurs combinaisons avec un vecteur pharmaceutiquement acceptable.

4. Utilisation d'une composition pharmaceutique selon l'une quelconque des revendications 1 ou 2 dans l'élaboration d'un médicament destiné au traitement prophylactique ou curatif de la maladie d'Alzheimer.

5. Utilisation selon la revendication 4, où ledit traitement consiste en l'administration quotidienne dudit médicament.
